# EUROPEAN PATENT SPECIFICATION

(11) **EP 3 060 301 B1**
(45) Date of publication and mention of the grant of the patent: **19.12.2018**
(21) Application number: 14856603.7
(22) Date of filing: 27.10.2014
(51) Int. Cl.: A61N 5/06

(54) **SYSTEMS AND METHODS FOR INCREASED VITAMIN D3 PRODUCTION**
SYSTEME UND VERFAHREN FÜR GESTEIGERTE VITAMIN-D3-PRODUKTION
SYSTÈMES ET PROCÉDÉS DE PRODUCTION ACCRUE DE VITAMINE D3

(30) Priority: 25.10.2013 US 201361895598 P
(43) Date of publication of application: 31.08.2016
(73) Proprietor: BeneSol, Inc., Bainbridge Island, WA 98110 (US)
(72) Inventor: MOFFAT, William A., Bainbridge Island, WA 98110 (US)
(74) Representative: Walker, Ross Thomson
(86) International application number: PCT/US2014/062352
(87) International publication number: WO 2015/061773

(56) References cited:
- WO-A1-2007/143862
- WO-A1-2007/143862
- WO-A2-2008/027438
- US-A1- 2005 143 793
- US-A1- 2006 106 435
- US-A1- 2008 224 592
- US-A1- 2011 004 280
- US-A1- 2013 172 963
- US-A1- 2013 172 963

## Description

### TECHNICAL FIELD

The present technology relates to vitamin D phototherapy, and more particularly to phototherapeutic systems and methods for enhanced vitamin D3 production.

### BACKGROUND

Vitamin D refers to a group of fat-soluble secosteriods that the human body can synthesize through adequate exposure to sunlight or UV radiation. More specifically, previtamin D3 is made in the skin when 7-dehydrocholesterol ("7-DHC") reacts with ultraviolet B ("UVB") light. Vitamin D can also be absorbed from the various dietary sources, such as fatty fish (e.g., salmon and tuna), vitamin D fortified foods (e.g., dairy and juice products), and vitamin D supplements. Once absorbed, the vitamin D travels through the bloodstream to the liver where it is converted into the prohormone calcidiol. The calcidiol is, in turn, converted into calcitriol (the hormonally active form of vitamin D) by the kidneys or monocyte-macrophages in the immune system. When synthesized by the monocyte-macrophages, calcitriol acts locally as a cytokine to defend the body against microbial invaders. Kidney-synthesized calcitriol circulates through the body to regulate the concentration of calcium and phosphate in the bloodstream, and thereby promotes adequate mineralization, growth, and reconstruction of the bones. Therefore, an inadequate level of vitamin D, (typically characterized by a calcidiol concentration in the blood of less than 20-40 ng/m²) can cause various bone softening diseases, such as rickets in children and osteomalacia in adults. Vitamin D deficiency has also been linked to numerous other diseases and disorders, such as depression, heart disease, gout, autoimmune disorders, and a variety of different cancers.

Physicians have recommended vitamin D supplements as a preventative measure to increase vitamin D levels. The American Institute of Medicine, for example, recommends a daily dietary vitamin D intake of 600 international units (IU) for those 1-70 years of age, and 800 IU for those 71 years of age and older. Other institutions have recommended both higher and lower daily vitamin D doses. The limitations on daily dosages also reflect an effort to prevent ingesting too much vitamin D, which can eventually become toxic. In contrast, the human physiology has adapted to significantly higher daily doses of vitamin D from sunlight (e.g., 4,000-20,000 IU/day or more). UVB radiation has been identified as a more desirable source of vitamin D because of the ease at which vitamin D is produced from exposure to sunlight and the body's natural ability to inhibit excessive vitamin D intake through the skin.

The International Commission on Illumination (also known as Le Commission Internationale de I'Eclairage ("CIE")) has created two standardized action spectrums associated with UV radiation and vitamin D production: "The Erythema Reference Action Spectrum and Standard Erythema Dose" (ISO 7166:1999), used to determine erythema (i.e., sunburn) response to individual wavelengths from 250 nm to 400 nm; and "The Action Spectrum for the Production of Previtamin D3 in Human Skin" (CIE 174:2006), used to determine the conversion efficiency of 7-DHC to previtamin D3 at individual wavelengths from 255 nm to 320 nm. After 7-DHC is converted to previtamin D3, it may be photoisomerized to either of two inert products, lumisterol or tachysterol, or it can undergo a reverse reaction and revert back to 7-DHC. These photoreactions are driven by continued UV radiation, but the absorption spectra of each photoproduct varies. A study used to create the CIE previtamin D3 action spectrum standardized the UV dosage to limit the conversion of 7-DHC to previtamin D3 to less than 5% to help mitigate any photoisomerization of previtamin D3 to photoproducts (e.g., lumisterol, tachysterol, and 7-DHC).

US 2008/0224592 A1 relates to a fluorescent lamp for use in stimulating previtamin D3 production and associated methods of determining the efficiency thereof. WO 2008/027438 relates to discharge lamps for use in stimulating vitamin D production and associated methods of determining the efficiency thereof. US 2013/0172963 A1 relates to a phototherapeutic apparatus for focused UVB radiation and vitamin D synthesis. WO 2007/143862 A1 relates to a phototherapeutic apparatus for treating vitamin D deficiency.

### SUMMARY OF THE INVENTION

The present invention relates to a method of determining the efficacy of a radiation assembly according to claim 1. Any subject matter provided herein that falls outside the scope of the claims is provided for information purposes only.

### BRIEF DESCRIPTION OF THE DRAWINGS

Many aspects of the present disclosure can be better understood with reference to the drawings shown below. The components in the drawings are not necessarily to scale. Instead, emphasis is placed on illustrating the principles of the present disclosure.
Figures 1-9 are graphs illustrating irradiance curves for filtered UV sources having emissions focused at wavelengths ranging from 298 nm to 306 nm in accordance with an embodiment of the present technology.
Figure 10 is a graph illustrating the relative effectiveness of previtamin D production and erythema as a function of wavelength in accordance with the CIE action spectrums and the ratio therebetween.
Figure 11 is a graph illustrating the ratio between the CIE previtamin D production action spectrum and the CIE erythema action spectrum as a function of wavelength.
Figure 12 is a graph illustrating the percentage conversion of 7-DHC to previtamin D3, lumisterol, and tachysterol at preselected wavelengths after exposure to 100 mJ/cm² of energy.
Figure 13 is a graph illustrating the percentage conversion of 7-DHC to previtamin D3, lumisterol, and tachysterol at preselected wavelengths after exposure to 1 J/cm² of energy.
Figure 14 is a graph illustrating the total percentage of 7-DHC, lumisterol, tachysterol, and previtamin D3 formed after exposure to 100 mJ/cm² of energy at the preselected wavelengths of Figure 12.
Figure 15 is a graph illustrating the total percentage of 7-DHC, lumisterol, tachysterol, and previtamin D3 formed after exposure to 1 J/cm² of energy at the preselected wavelengths of Figure 13.
Figure 16 is a graph illustrating photoisomerization action spectrums for radiation sources emitting energy of 100 mJ/cm² and 1 J/cm² and the CIE previtamin D3 production action spectrum.
Figure 17 is a graph illustrating a vitamin D3 phototherapy action spectrum for 100 mJ/cm² of energy, the corresponding photoisomerization action spectrum of Figure 16, and the curve of Figure 11 representing the ratio between the CIE previtamin D production action spectrum and the CIE erythema action spectrum.
Figure 18 is a graph illustrating an action spectrum of a filtered radiation assembly with emissions centered at 302 nm configured in accordance with an embodiment of the present technology.
Figure 19 is a graph illustrating action spectrums of a radiation source with a plurality of different filters centered at different wavelength targets.
Figure 20 is an isometric view of a phototherapeutic system for focused UVB radiation configured in accordance with an embodiment of the present technology.

### DETAILED DESCRIPTION

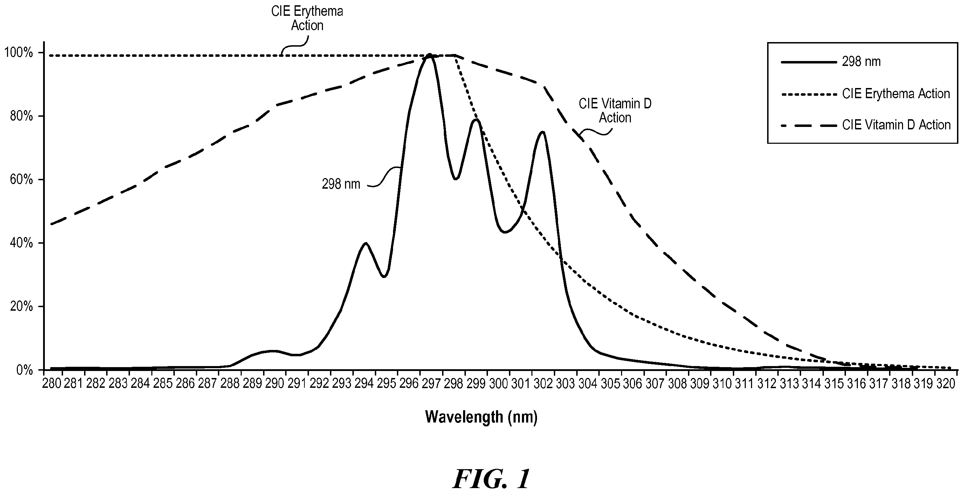

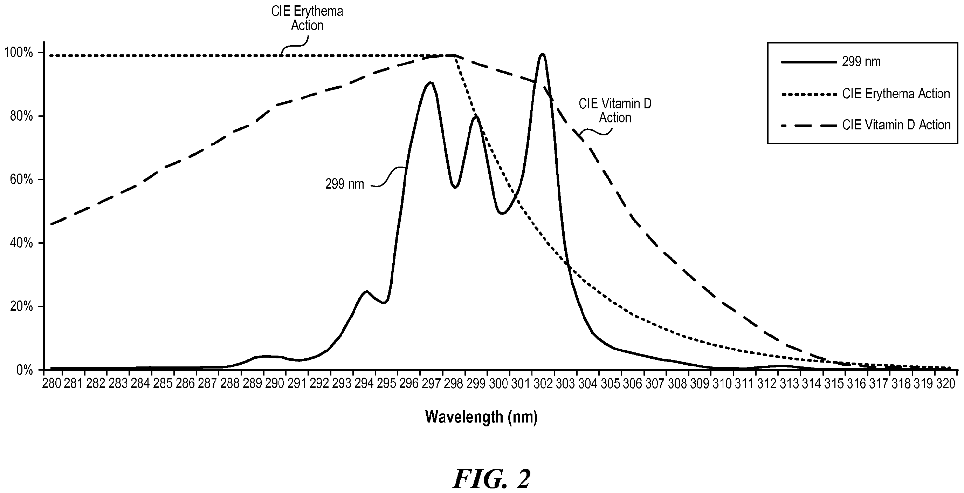

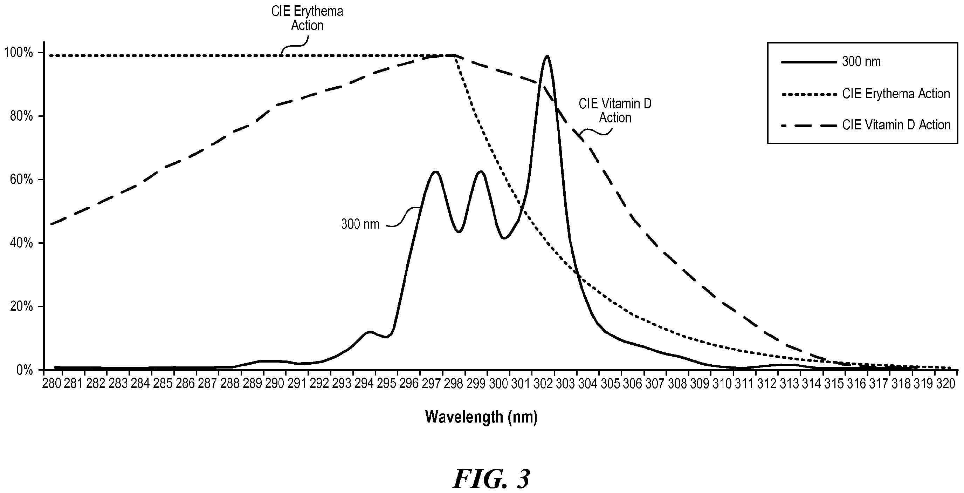

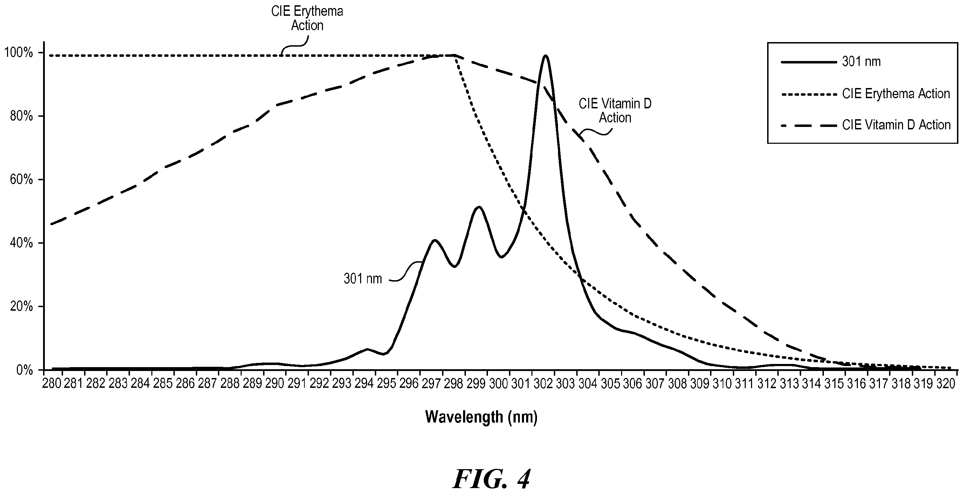

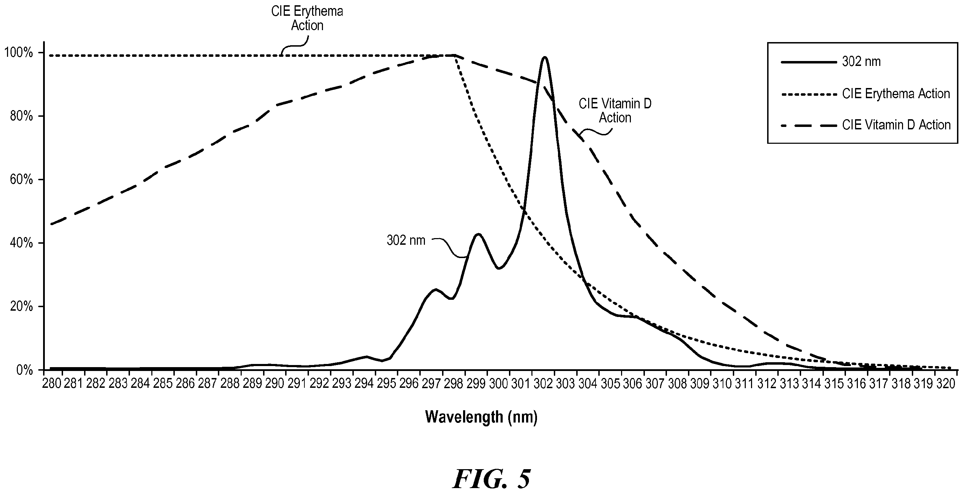

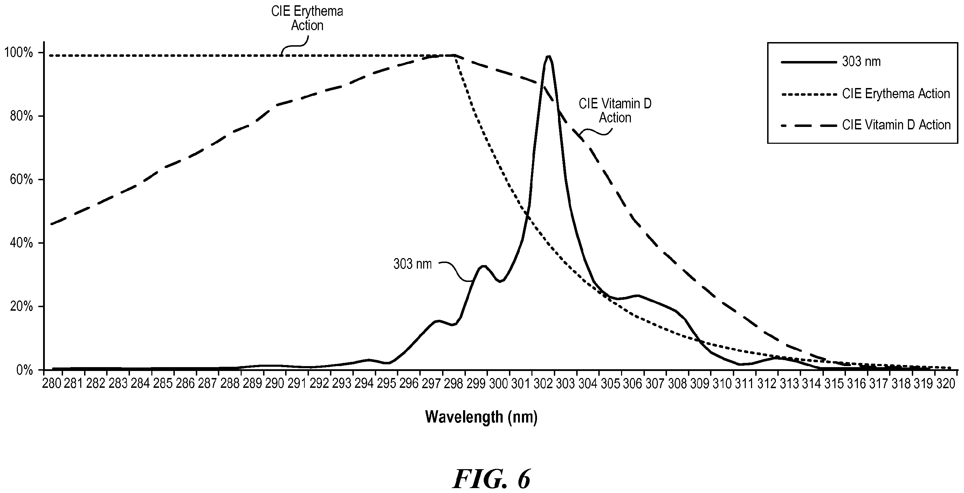

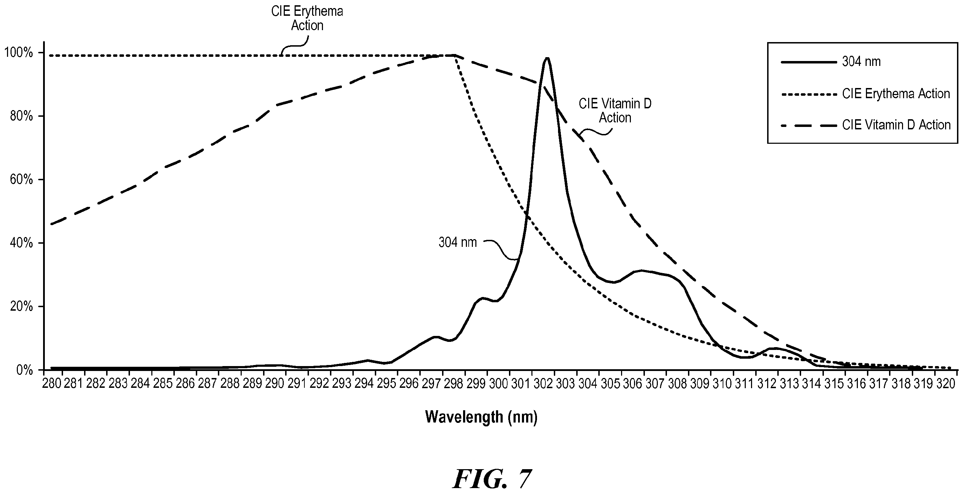

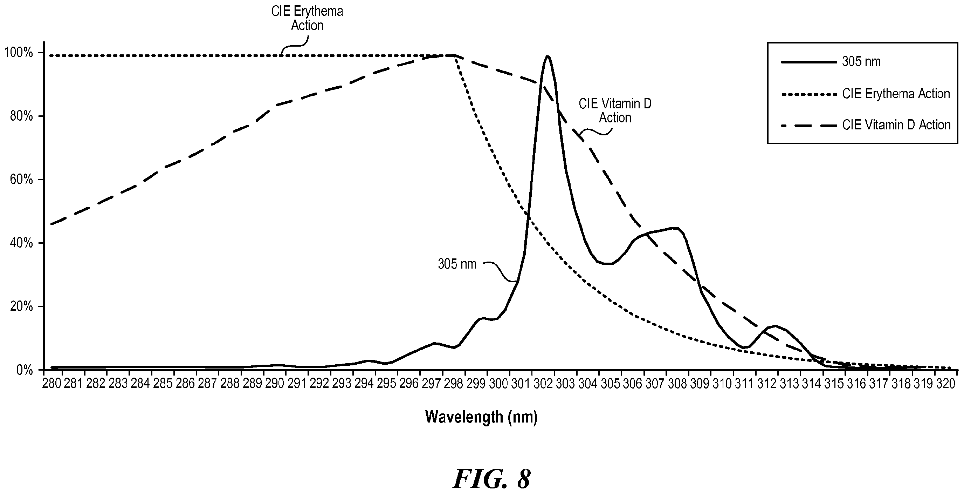

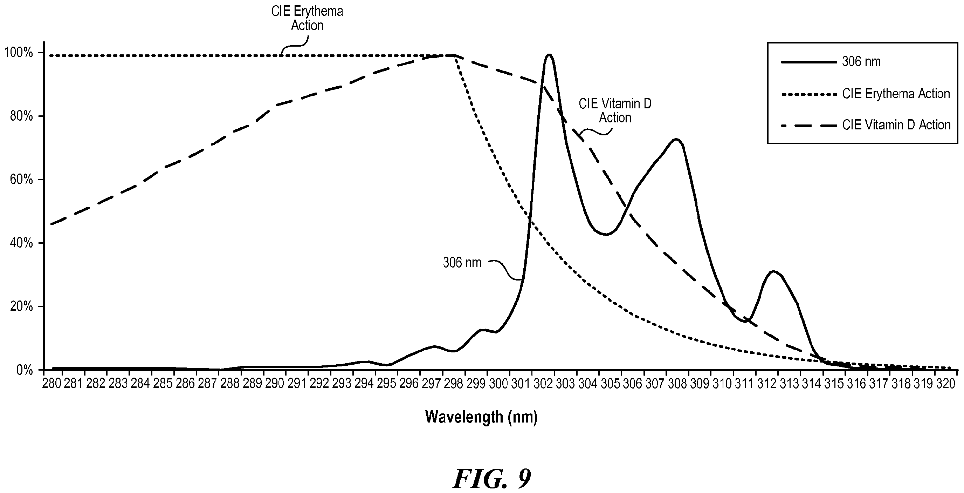

The present technology is directed to apparatuses, systems, and methods for providing an efficacious UVB wavelength range to achieve maximum vitamin D production in the skin during a single phototherapy treatment session with minimum UV exposure. Such apparatuses, systems, and methods can be based on a vitamin D3 phototherapy action spectrum, which has been developed using the processes and methods described below. Specific details of several embodiments are described below with reference to Figures 1-21. Although many of the embodiments are described below with respect to systems, devices, and methods for promoting vitamin D production in the skin, other applications (e.g., phototherapeutic treatment of psoriasis or skin diseases) in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below with reference to Figures 1-21.

### I. Selected Methods and Systems for Defining A Vitamin D3 Phototherapy Action Spectrum

The efficiency with which a certain wavelength of UV emissions produces previtamin D3 in the skin can be determined by first gathering irradiance data from UV sources or radiation assemblies focused at various desired wavelengths. For example, irradiance data can be gathered from UV sources that are filtered to emit radiation centered at about 298 nm to about 306 nm, or other ranges of wavelengths suitable for vitamin D production via the skin. As described in further detail below, the irradiance data from each filtered UV source can then be compared to each other and to the CIE previtamin D3 action spectrum and the CIE erythema action spectrum to determine the wavelength output that provides the most vitamin D production, while also limiting the amount of exposure to radiation that causes sunburn.

Figures 1-9, for example, are graphs illustrating irradiance curves for filtered radiation sources having emissions focused at various different wavelengths in accordance with embodiments of the present technology. In the illustrated graphs, irradiance data was taken from filtered radiation sources focused at wavelengths ranging from 298 nm to 306 nm, in 1 nm increments. This wavelength range is generally thought to suitable for vitamin D production. However, in other embodiments, irradiance data may be gathered from radiation assemblies focused at higher or lower wavelengths and/or measured at smaller or larger wavelength intervals.

The data illustrated in the graphs of Figures 1-9 was gathered from a UV source comprising a 150 W doped metal halide lamp with an integrating sphere attached to a spectroradiometer. Irradiance was measured, via the spectroradiometer, from 250 nm to 400 nm, with a resolution of 1 nm. In other embodiments, irradiance data can be gathered from different types of UV sources, such as light emitting diodes ("LEDs"), excimer lamps, and/or pulse xenon lamps, and/or from different spectral ranges. Various filters, such as interference coatings, can be used in conjunction with the UV source to focus the emissions around a target wavelength. For example, a multi-layer vapor deposition interference coating can be applied to a quartz substrate material to achieve a UV narrow-pass transmission range that is centered on a target wavelength with a width of +/- 4 nm. In other embodiments, interference coatings may be applied to other suitable substrates for UV radiation, disposed on the substrate using other suitable deposition means, and/or have a larger or narrower bandwidth (e.g., 10 nm, 12 nm, 16 nm, etc.). Filter properties can also be simulated via computer programs known in the art. For example, the graphs illustrated in Figures 1-9 were generated using irradiance data measured from the 150 W metal halide lamp, in combination with simulated interference coatings with target wavelength of 298 nm to 306 nm, to provide a series of theoretical spectral analysis datasets.

As further shown in Figures 1-9, the datasets gathered from the filtered UV sources (e.g., via direct measurement and/or simulation) can be compared to the two CIE action spectrums (i.e., the CIE erythema action spectrum and the CIE previtamin D action spectrum). As shown in Figure 1, maximum D3 production with minimum UV exposure occurs at the intersect of the two CIE action spectrums, when the target wavelength of the filter (e.g., interference coating) is focused at 298 nm (keeping exposure time constant).

However, a target wavelength of 298 nm does not necessarily maximize vitamin D3 production per treatment when the length of the treatment is variable based on a constant minimal erythemal dose ("MED"). The MED is the amount of UV radiation that will produce minimal erythema (i.e., sunburn or redness caused by engorgement of capillaries) of an individual's skin within a few hours following exposure. The MED can be determined using the CIE erythema action spectrum (i.e., the curve shown in Figures 1-9) as a weighting factor for spectral irradiance output from a UV source.

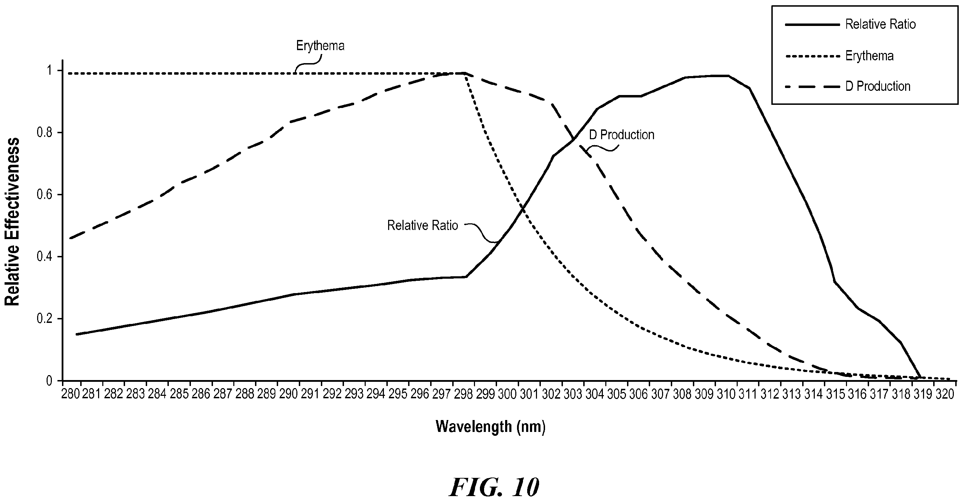

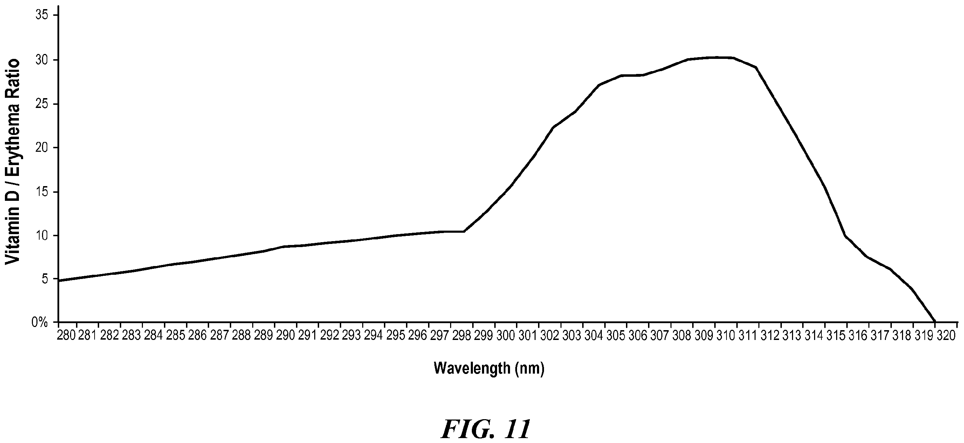

In various embodiments, the duration of UV exposure during a phototherapy session can be prescribed according to an individual's skin sensitivity. When the treatment time is selected based on a constant MED dose response, the amount of vitamin D produced per treatment is significantly impacted by the ratio between the CIE erythema action spectrum and the CIE previtamin D3 action spectrum. Accordingly, it is expected that maximizing the ratio of CIE previtamin D3 production to CIE erythema (D3:erythema) will maximize previtamin D3 production during a phototherapy session that is limited by the MED. That is, a higher ratio between previtamin D3 production and erythema allows a higher dose of UV per treatment without causing reddening of the skin, and therefore increases total vitamin D3 production per treatment session. The graph shown in Figure 10 illustrates the CIE previtamin D production and CIE erythema curves, as well as a curve illustrating the ratio between them (identified as "Relative Ratio"). The graph of Figure 11 shows the curve of the ratio between CIE previtamin D production and erythema. As shown in Figures 10 and 11, the greatest D3:erythema ratio occurs at about 309 nm. More specifically, as shown in Figure 11, the ratio of vitamin D production to erythema is about 30 at 309 nm.

As noted above, previtamin D3 may revert back to 7-DHC or undergo photoisomerization into inert photoproducts during continued exposure to UV radiation. Accordingly, in order to increase or maximize vitamin D production during a single phototherapy session, the conversion of previtamin D3 back to 7-DHC and other photoproducts as more UV radiation is administered should be reduced or minimized. Experiments can be performed to determine the wavelength or wavelengths that provide maximum previtamin D3 production and minimum photoisomerization of previtamin D3 to photoproducts . For example, a solution of 7-DHC (i.e., the precursor to previtamin D3) can be housed in a sealed ampule or container and exposed to a UV source (e.g., a tunable laser or monochromator). The UV source can apply a constant energy to the 7-DHC samples, and can be tuned to varying monochromatic radiation wavelengths, such as from 290 nm to 308 nm. For example, in certain embodiments samples of a 7-DHC solution are exposed to 100 mJ/cm² of energy at individual wavelengths of 290 nm, 292 nm, 294 nm, 295 nm, 296 nm, 298 nm, 300 nm, 302 nm, 304 nm, 306 nm, 308 nm. The same process can be repeated at the selected wavelengths for one or more other energy levels, such as 1,000 mJ/cm². In other embodiments, samples of 7-DHC can be exposed to tunable lasers or other UV radiation devices tuned to different energy levels and/or different wavelengths. After radiation exposure to the preselected wavelengths, the contents of each ampule of the 7-DHC solution can be measured to determine the amount of 7-DHC, previtamin D3, lumisterol and tachysterol present in the sample.

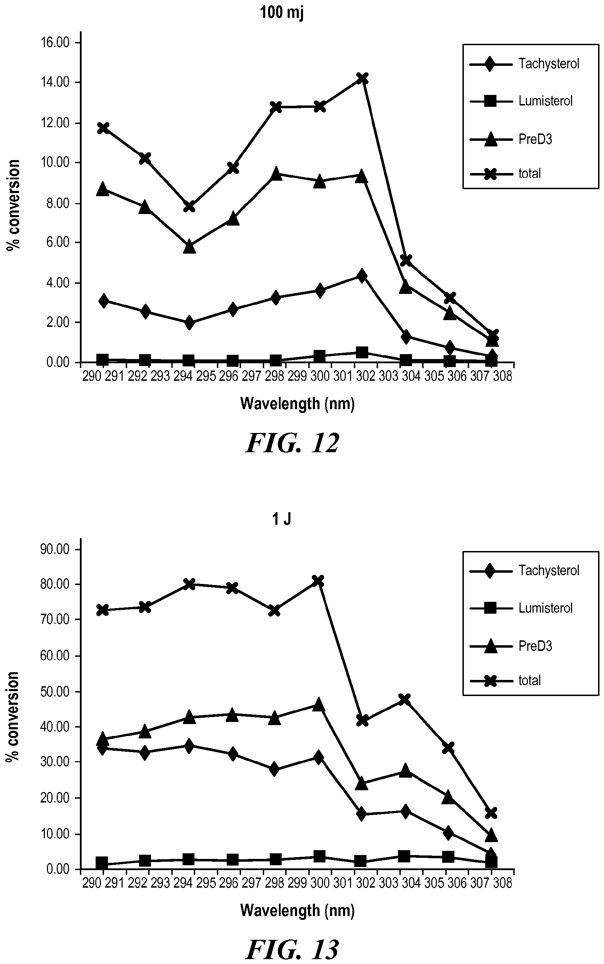

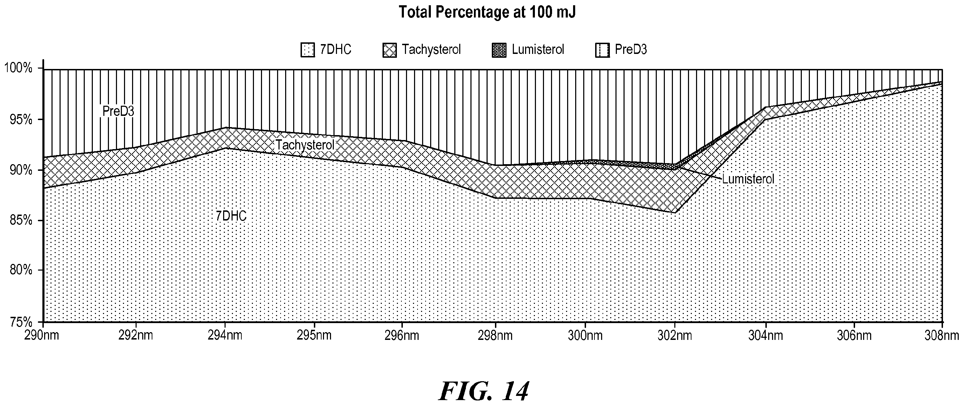

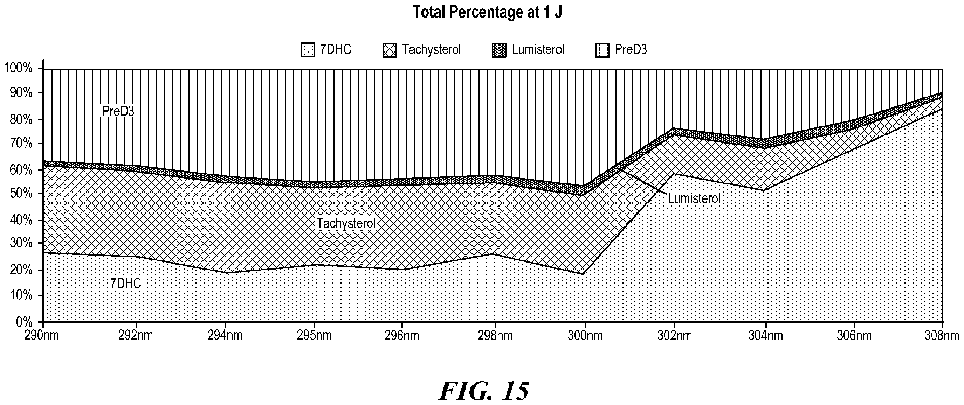

Figures 12 and 13 are graphs illustrating raw data of the percentage of the 7-DHC converted to previtamin D3, tachysterol, and lumisterol measured from the samples described above. More specifically, the graphs illustrate the conversion of 7-DHC to previtamin D3, lumisterol, and tachysterol at preselected wavelengths for a radiation source tuned to emit 100 mJ/cm² of energy (Figure 12) and for a radiation source tuned to emit 1 J/cm² of energy (Figure 13). The graphs of Figures 14 and 15 illustrate the total percentage of 7-DHC, lumisterol, tachysterol, and previtamin D3 in each specimen at the preselected wavelengths after exposure to 100 mJ/cm² of energy (Figure 14) and 1 J/cm² of energy (Figure 15). As shown in Figures 14, for a radiation source energy of 100 mJ/cm², the maximum previtamin D3 conversion with minimum photoisomerization of previtamin D3 to photoproducts (i.e., 7-DHC, lumisterol, and tachysterol) occurs at wavelengths ranging from about 298 nm to 302 nm. For a radiation source energy of 1,000 mJ/cm², the wavelength for minimum photoproducts is about 300 nm.

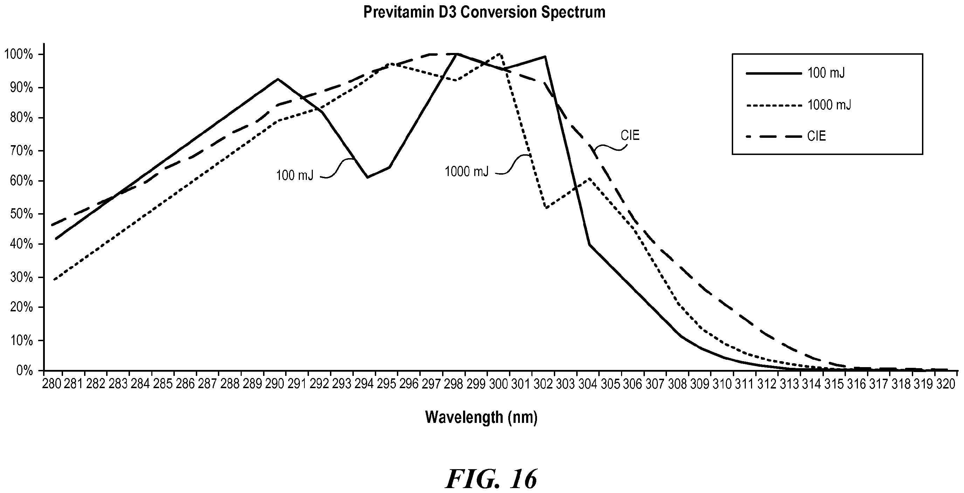

This photoproduct conversion information can be used to create photoisomerization action spectrums for the selected energy levels, which can then be compared with the CIE previtamin D3 production action spectrum. Figure 16, for example, is a graph illustrating the photoisomerization action spectrums at 100 mJ/cm² and 1,000 mJ/cm², with the CIE vitamin D3 production action spectrum superimposed thereon. As shown in Figure 16, while the CIE vitamin D3 action spectrum indicates that maximum previtamin D3 production occurs at wavelengths of about 297 nm to 298 nm, the photoisomerization action spectrums indicate that wavelengths of about 300 nm to 302 nm would allow greater previtamin D3 preservation after initial production. That is, radiation with wavelengths of about 300-302 nm causes lower levels of photoproducts(i.e., 7-DHC, lumisterol, and tachysterol) to form during UV radiation, and therefore allows more previtamin D3 to be formed and maintained so it can actually be used in the previtamin D3 form by the body. Accordingly, providing radiation at wavelengths of about 300-302 nm is expected to allow for greater vitamin D production and greater energy delivery during a single phototherapy treatment than could be obtained using a lower wavelength range (e.g., focused at about 298 nm).

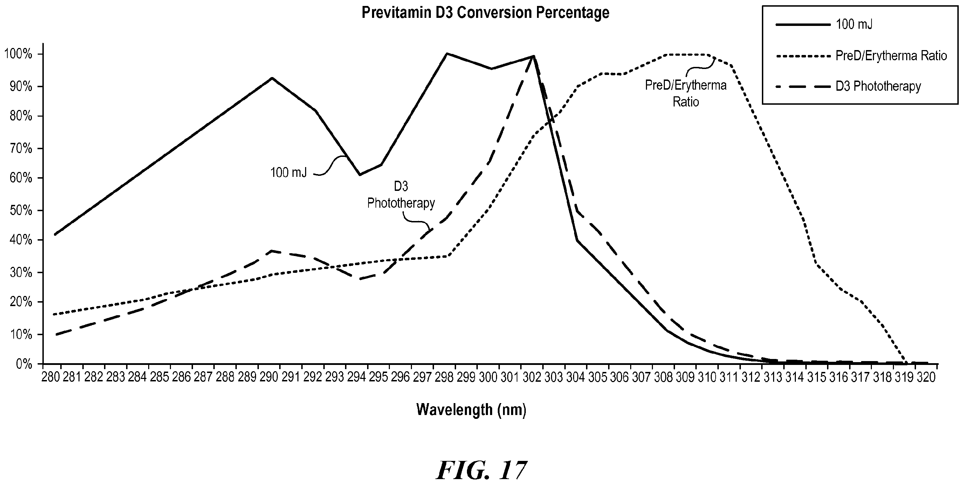

This information can then be used to create an action spectrum for maximum vitamin D3 production per phototherapy treatment session. For example, the vitamin D3 phototherapy action spectrum can be constructed by combining three action spectrums: the CIE previtamin D3 production action spectrum, the CIE erythema action spectrum, and the newly-created action spectrum that exhibits the minimum photoisomerization of previtamin D3 to photoproducts for a given energy level (e.g., as shown in Figure 16). Further, as described above, in certain embodiments phototherapy sessions can be standardized by MED. Accordingly, in these embodiments the previtamin D3/erythema ratio action spectrum shown in Figure 11 can be used to represent the two CIE action spectrums. Assuming that the amount of energy delivered during a typical phototherapy session is less than 100 mJ/cm², the 100 mJ/cm² action spectrum for minimum photoproduct conversion shown in Figure 16 can be used in the algorithm. The previtamin D3/erythema ratio at each wavelength can be multiplied by the minimum photoproduct conversion at each wavelength for the given energy level (i.e., 100 mJ/cm²) to construct the vitamin D3 phototherapy action spectrum shown in the graph of Figure 17 (identified as "D3 Phototherapy"). In embodiments, such as when the energy delivered is 1 J/cm² or more, a different photoproduct conversion curve can be multiplied with the previtamin D3/erythema ratio to determine the vitamin D3 phototherapy action spectrum for that energy level.

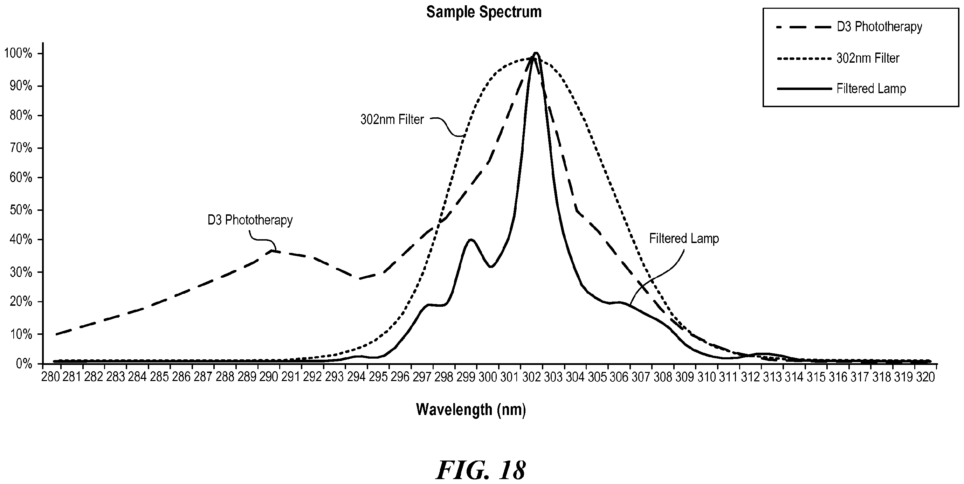

The vitamin D3 phototherapy action spectrum provides a single calculation of a spectrum analysis that determines the effectiveness of a radiation source and/or filtration system so that a phototherapy session can produce maximum levels of vitamin D3 production in the skin with minimum total UV exposure. In practice, the vitamin D3 phototherapy action spectrum allows radiation sources and/or radiation assemblies with filters to be rated by their relative efficacy. For example, the irradiance values for each wavelength of a radiation source can be multiplied by the efficacy percentage for each wavelength on the vitamin D3 phototherapy action spectrum of Figure 17, and thereby provide a weighted irradiance value. The weighted irradiance values for each wavelength can then be totaled and divided by the total of the unweighted irradiance values for each wavelength. According to the vitamin D3 conversion action spectrum at a 100 mJ/cm² exposure (Figures 16 and 17), a perfect relative efficacy of 100% occurs using a monochromatic UV source with all radiation emitted at a wavelength of 302 nm. Figure 18 is a graph illustrating a spectrum of a metal halide lamp filtered by a narrow pass (+/- 4 nm) interference coating with a 302 nm center target (spectrum of filtered lamp identified as "Filtered Lamp"; spectrum of filter identified as "302nm Filter"). Comparing the filtered lamp spectrum to the vitamin D3 phototherapy action spectrum (identified as "D3 Phototherapy" in Figure 18) using the algorithm described above, the relative efficacy of the filtered lamp is 64.58%. In other embodiments, the spectrum of UV assemblies having different UV sources and/or filters can be compared to the D3 phototherapy action spectrum for 100 mJ./cm² to determine the efficacy of the radiation assembly and increase or maximize the vitamin D3 production during a single phototherapy session. Standardized D3 phototherapy action spectrums can be defined for different energy levels so that the most efficient radiation sources can be selected for a given energy level.

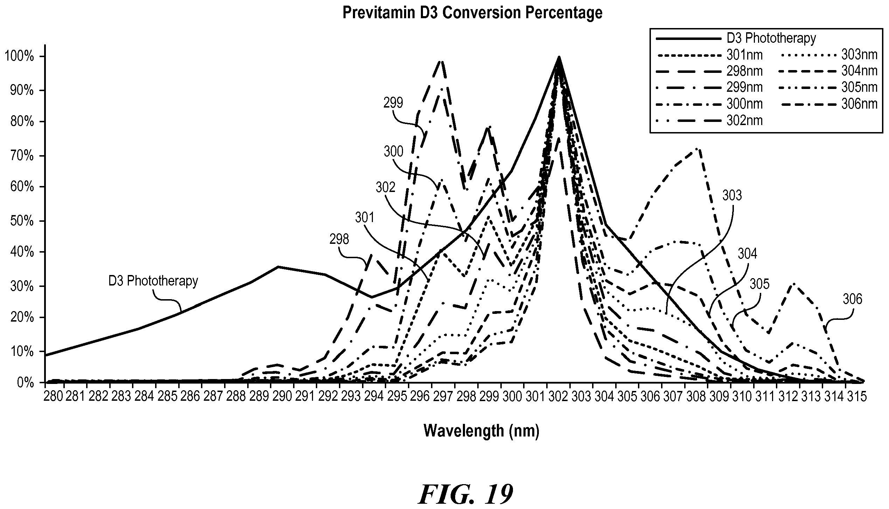

Accordingly, the vitamin D3 phototherapy action spectrum can be used as a tool to analyze radiation sources and/or different filter and radiation source combinations. This allows manufacturers to consider the efficacy of radiation assemblies (e.g., including UV sources and, optionally, filters) when designing phototherapy apparatuses. For example, Figure 19 is a graph illustrating the action spectrums of radiation sources (e.g., doped metal halide lamps) with filters focused at numerous different wavelength targets (i.e., 301-306 nm). The data for each action spectrum can be multiplied by the vitamin D3 phototherapy action spectrum at the corresponding wavelength to determine the efficacy of each filtered radiation assembly, and the most efficient and/or most cost effective radiation assembly can be selected for a phototherapy system. For example, the action spectrum of the filtered lamp shown in Figure 18 was determined to be the most efficacious after analyzing action spectrums of a doped metal halide lamp filtered by various different interference coatings centered at different wavelength targets. Accordingly, the processes described above are expected to enhance the efficacy of phototherapy sessions by providing increased or maximized vitamin D production and reduced erythema during each phototherapy session.

### II. Selected Embodiments of Phototherapeutic Systems

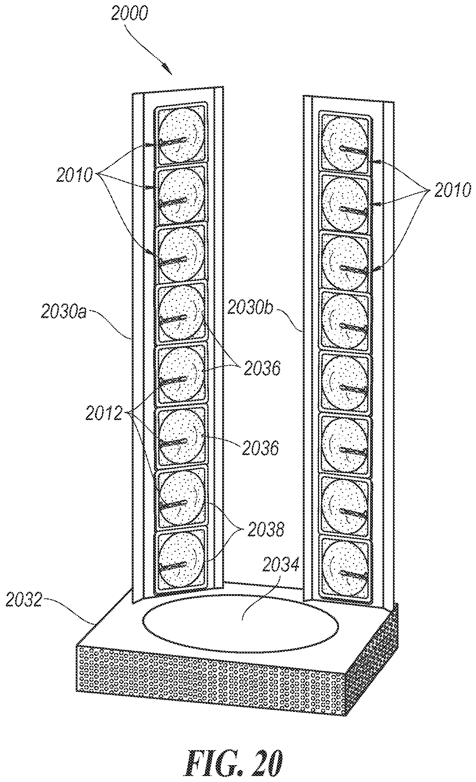

Figure 20 is an isometric view of a phototherapeutic apparatus or system ("system 2000") for focused UV radiation configured in accordance with an embodiment of the present technology. The system 2000 includes a plurality of focused UV radiation fixtures or assemblies 2010 ("radiation assemblies 2010") that emit energy within a predetermined wavelength range (e.g., about 300-304 nm, 298-302 nm, etc.). In the illustrated embodiment, the radiation assemblies 2010 are carried by two housings, arms or columns (identified individually as a first column 2030a and a second column 2030b, and referred to collectively as columns 2030) that are mounted on or otherwise attached to a pedestal or base 2032, and the radiation assemblies 2010 are directed generally inward toward a central portion 2034 of the base 2032. The base 2032 and the columns 2030 together define an irradiation zone in which a human can be exposed to focused UVB energy emitted by the radiation assemblies 2010. When a user (e.g., a human) stands on or is otherwise positioned at the central portion 2034 of the base 2032, the radiation assemblies 2010 can irradiate the user's skin to stimulate vitamin D production in the skin during a phototherapy session. In various embodiments, the central portion 2034 of the base 2032 and/or the columns 2030 may rotate relative to each other to expose all sides of the user's body to the energy emitted by the radiation assemblies 2010.

In the embodiment illustrated in Figure 20, the system 2000 includes eight radiation assemblies 2010 in each column 2030 that emit energy at substantially similar wavelengths and similar intensities. In certain embodiments, the radiation assemblies 2010 in the first column 2030a can be vertically offset from the radiation assemblies 2010 in the second column 2030b to prevent the irradiation from radiation assemblies 2010 of the first column 2030a from directly overlapping the irradiation from the radiation assemblies 2010 of the second column 2030b. For example, the radiation assemblies 2010 in the first column 2030a can be offset from radiation assemblies 2010 in the second column 2030b by about one radius of an individual radiation assembly 2010. This staggering of the radiation assemblies 2010 can provide a more uniform intensity of irradiation along the length of the columns 2030 and prevent certain areas of a user's skin from being exposed to more irradiation than others. In other embodiments, the system 2000 can include different features and/or other radiation assembly configurations to enhance the uniformity of the radiation emitted by the radiation assemblies 2010 and/or manipulate the direction in which the radiation is projected. For example, one or more lenses can be positioned forward of one or more of the radiation assemblies 2010 and configured to bend the light in a manner such that the light is evenly distributed across the irradiation zone or a portion thereof. In further embodiments, the system 2000 can include columns 2030 with fewer than or more than eight radiation assemblies 2010 (e.g., one radiation assembly, two radiation assemblies, four radiation assemblies, nine radiation assemblies, etc.), a single column 2030 of radiation assemblies 2010, more than two columns 2030 of radiation assemblies 2010 (e.g., four columns, six columns, etc.), and/or the radiation assemblies 2010 can be arranged in other suitable configurations. For example, the radiation assemblies 2010 can be carried by a housing that at least substantially encloses the irradiation zone and directs radiation inward toward an enclosed space defined by the housing.

The system 2000 can emit high intensity focused UVB radiation to facilitate vitamin D production in the skin during relatively short phototherapy sessions. For example, the apparatus 2000 can provide a sufficient amount of irradiation during a phototherapy session (e.g., 30 seconds, 1 minute, 2 minutes, 5 minutes, etc.) to stimulate the production of a weekly or monthly dose of vitamin D. In various embodiments, the exposure time of each phototherapy session can be selected based on the on the user's skin type (e.g., as defined by the Fitzpatrick scale) and/or the intensity of the radiation assemblies 2010. For example, the lighter the user's skin tone, the less exposure time necessary to obtain the desired level of vitamin D synthesis in the user's skin or the less exposure time allowed to avoid overexposing the user's skin. As another example, the higher the intensity of the energy provided by the system 2000, the less exposure time necessary to obtain the desired irradiation for vitamin D production. In further embodiments, the duration of the phototherapy sessions can also be selected to at least reduce the likelihood that users experience sunburn after the phototherapy session. For example, the exposure time to UVB irradiation can be limited to a user-specific MED of 1.0 or less (e.g., a MED of 0.75). In other embodiments, the exposure time of system 2000 can be determined using the standardized MED and/or other suitable parameters for UVB irradiation and/or vitamin D synthesis.

As shown in Figure 20, each radiation assembly 2010 can include a UV radiation source 2012, a reflector 2036 partially surrounding the UV radiation source 2012, and a filter 2038 forward of the radiation source 2012. The radiation source 2012 can emit energy (e.g., UV light), and at least some of the energy can contact the reflector 2036 (e.g., a mirrored substrate or coating) before exiting the radiation assembly 2010. The reflector 2036 can divert or otherwise direct the light forward toward the filter 2038 where light within a predetermined bandwidth (e.g., 6 nm, 8 nm, 16 nm, etc.) can exit the radiation assembly 2010. In certain embodiments, the reflector 2036 is curved around the radiation source 2012 such that the light emitted by the radiation source 2012 collimates upon contact with the reflector 2036. The collimated beam of light can then travel forward toward the filter 2038, and pass through the filter 2038 at the same angle of incidence (e.g., 0°) to provide substantially uniform filtering of the light. In other embodiments, the radiation assemblies 2010 may not include the reflector 2036, and/or the radiation assemblies 2010 can include other features that collimate the radiation emitted from the radiation sources 2012.

The radiation source 2012 can include a metal halide lamp, which is a type of high-intensity discharge ("HID") lamp that generates light by producing an electric arc through a gaseous mixture between two electrodes in an arc tube or envelope. The arc length (i.e., about the distance between the electrodes) of the metal halide lamp can be relatively small with respect to radiation assembly 2010 as a whole such that the metal halide lamp acts similar to a point source to facilitate collimation of the light In other embodiments, the metal halide lamp can have larger or smaller arc lengths depending on the configuration of the metal halide lamp and the sizing of the other components of the radiation assembly 2010 (e.g., the reflector 2036).

In various embodiments, the gas mixture in the arc tube of the metal halide lamp can be selected to increase the UVB content of the emissions of the metal halide lamp. For example, the gas mixture can be doped to generate about 6% of the total emissions in the UVB range (e.g., about 280-315 nm) in comparison to normal tanning bed lamps that have about 1% of their emissions in the UVB range. The increased UVB content of the emissions can increase the intensity of the UVB emitted by the radiation assembly 2010, and therefore may decrease the overall exposure time necessary to achieve a desired vitamin D dose. Based on test data, it is believed that large portions of the emissions of doped metal halide lamps have wavelengths of about 300-305 nm. As discussed above with respect to Figures 16-18, the D3 phototherapy action spectrum suggests that 302 nm is an optimal wavelength for maximum previtamin D3 production and minimized erythema for radiation concentrations of less than 1,000 mJ/cm². Accordingly, metal halide lamps are uniquely suited for promoted vitamin D production in the skin and may require less filtering than other types of UV radiation sources.

The filter 2038 can be a narrow pass filter that prevents UVB radiation outside of a predetermined bandwidth from exiting the radiation assembly 2010. In certain embodiments, the filter 2038 can include a substrate (e.g., glass, plastic, etc.) and at least one interference coating applied to the substrate. The coating can be sprayed onto the substrate and/or otherwise disposed on the substrate using methods known to those skilled in the art. Substrates and interference coatings that provide at least some filtering of UV radiation outside of a predetermined spectrum are available from Schott of Elmsford, New York. In various embodiments, other portions of the radiation assemblies 2010 can include interference coatings and/or other filtering features that block at least some radiation outside of the desired wavelength spectrum. For example, an absorption filter can be incorporated into the envelope of a metal halide lamp (e.g., metal additives can be incorporated into the quartz of the lamp). The vitamin D3 phototherapy action spectrum described above can be used to determine the most efficient wavelength for the vitamin D production for a given radiation source, and a narrow pass filter can be designed or selected to emit radiation centered at the predetermined wavelength. For example, in certain embodiments, the filter 2038 can at least substantially block UVB radiation outside of a 4 nm spectrum centered at about 302 nm (i.e., about 300-304 nm) or a 10 nm spectrum centered at about 300 nm (i.e., about 295-305 nm). In other embodiments, the filter 2038 can at least substantially block UVB radiation outside of a different bandwidths (e.g., a 6 nm spectrum, an 8 nm spectrum, a 12 nm spectrum, a 16 nm spectrum, etc.), and/or the spectrum can be centered around other suitable wavelengths for vitamin D production (e.g., 298 nm, 300 nm, 302 nm, etc.).ln other embodiments, the system 2000 can include other types of UV radiation sources that, in combination with optional filters, can provide focused UVB irradiation within a predetermined spectrum. For example, a UV radiation source can be comprised of a plurality of LEDs (e.g., thousands of LEDs) that emit light at a particular wavelength (e.g., 295 nm, 297 nm, 300 nm, 302 nm, 304 nm, etc.). Suitable LEDs are available from, for example, Sensor Electronic Technology, Inc. of Columbus, South Carolina. The substantially monochromatic output of the LEDs may reduce or eliminate the amount of filtering necessary to provide UVB radiation within a predetermined spectrum, In further embodiments, the UV radiation source can be comprised of excimer lamps that can emit light within a narrow spectral range and/or other suitable UV radiation sources that can be filtered or otherwise manipulated for focused UVB radiation.

The concentrated UVB radiation provided by the system 2000 can deliver a large dose of vitamin D (e.g., a weekly dose, a monthly dose, etc.) to the user within a relatively short phototherapy session (e.g., less than 10 minutes, less than 5 minutes, less than 2 minutes, less than 1 minute, etc.) in comparison to the length of sun exposure necessary to produce the same amount of vitamin D. The radiation sources 2012 and narrow bandwidth filters 2038 can be selected based on the vitamin D3 action spectrum described above (e.g., as shown in Figure 18). Using the vitamin D3 phototherapy action spectrum as guidance, the system 2000 can include one or more radiation assemblies that provide an increased or maximum level of vitamin D production for an MED, and therefore provide efficient phototherapy treatments.

### III. Examples

The following Examples are illustrative of several embodiments of the present technology.
1. A method for enhancing vitamin D3 production during a phototherapy session, the method comprising:
   measuring irradiance data from a radiation assembly focused at a target wavelength;
   multiplying irradiance values at a selected range of wavelengths between 280 nm and 320 nm with efficacy values of a vitamin D3 phototherapy action spectrum at the corresponding wavelengths to determine a weighted irradiance value at each wavelength, wherein the phototherapy action spectrum defines a wavelength having maximum vitamin D production per minimal erythemal dose at a predetermined energy level;
   summing the weighted irradiance values to determine a total weighted irradiance value;
   dividing the total weighted irradiance value by a total of the irradiance values at the selected range of wavelengths to determine the efficiency of the radiation assembly; and
   delivering, via the radiation assembly, ultraviolet rays focused at the target wavelength to a human to stimulate vitamin D production during the phototherapy session, wherein a duration of the phototherapy session is limited to a minimum erythemal dose.
2. The method of example 1, further comprising forming the vitamin D3 phototherapy action spectrum at the predetermined energy level, wherein forming the vitamin D3 phototherapy action spectrum comprises:
   determining a percentage of photoproduct conversion for the predetermined energy level across a spectrum of wavelengths; and
   multiplying the photoproduct conversion at a plurality of wavelengths with a ratio of CIE previtamin D3 production to CIE erythema action spectrum at the corresponding wavelengths, wherein the vitamin D3 phototherapy action spectrum for the predetermined energy level corresponds to a curve associated with the multiplied values at each wavelength.
3. The method of example 2, further comprising:
   measuring photoproduct conversion of a plurality of samples of 7-DHC exposed to the predetermined energy level at a corresponding plurality of wavelengths, wherein the photoproduct conversion measures quantities of previtamin D3, lumisterol, tachysterol, and 7-DHC in the samples of 7-DHC after exposure to the predetermined energy level; and
   defining a photoisomerization action spectrum for the predetermined energy level, wherein the photoisomerization action spectrum defines the percentage of photoproduct conversion.
4. The method of any one of examples 1-3 wherein the predetermined energy level is at most 1 J/cm².
5. The method of any one of examples 1-4 wherein the vitamin D3 phototherapy action spectrum is standardized by minimum erythemal dose.
6. The method of any one of examples 1-5 wherein:
   measuring irradiance data from the radiation assembly comprises measuring irradiance data for a plurality of radiation assemblies, each radiation assembly being focused at a different target wavelength; and
   the method further comprises determining the efficiency of each radiation assembly by performing the steps of multiplying, summing and dividing for each radiation assembly.
7. The method of any one of examples 1-6 wherein the target wavelength is between 300 nm and 302 nm.
8. The method of any one of examples 1-7 wherein the radiation assembly comprises a metal halide lamp and a filter, the filter comprising an interference coating on a substrate, wherein the interference coating has a bandwidth of at most 16 nm.
9. The method of any one of examples 1-8, further comprising a determining minimum erythemal dose of the radiation assembly by weighting irradiance values at a selected wavelength with a CIE erythema action spectrum at the selected wavelength.
10. A phototherapeutic system, comprising:
   an ultraviolet (UV) source directed toward an irradiation zone, wherein the UV source is configured to deliver a predetermined energy level during a phototherapy session; and
   a filter between the UV source and the irradiation zone, the filter being configured to at least substantially remove UV radiation outside of a predetermined wavelength spectrum, wherein the predetermined spectrum has a bandwidth of at most 16 nm and is focused at a wavelength corresponding to a maximum on a vitamin D3 phototherapy action spectrum for the predetermined energy level.
11. The phototherapeutic system of example 10 wherein:
   the UV source comprises a metal halide lamp; and
   the filter comprises an interference coating.
12. The phototherapeutic system of example 10 or 11 wherein the phototherapeutic system is configured to maximize previtamin D3 production per minimum erythemal dose, and further configured to minimize photoisomerization of vitamin D3.
13. The phototherapeutic system of any one of examples 10-12 wherein the predetermined energy level is at most 1 J/cm².
14. The phototherapeutic system of any one of examples 10-13 wherein the filter is focused at a target wavelength of 300-302 nm.
15. The phototherapeutic system of any one of examples 10-14 wherein the filter comprises an interference coating with a bandwidth of at most 8 nm centered at 302 nm.
16. The phototherapeutic system of any one of examples 10-15 wherein the vitamin D3 phototherapy action spectrum is defined by the product of a photoisomerization action spectrum for the predetermined energy level across a plurality of wavelengths and a ratio of CIE previtamin D3 production to CIE erythema action spectrum at the corresponding wavelength.
17. The phototherapeutic system of any one of examples 10-16 wherein the UV source and the filter define one of a plurality of radiation assemblies, and wherein the phototherapeutic system further comprises a base carrying the radiation assemblies, wherein the radiation assemblies are directed generally inward toward a central portion of the base to define the irradiation zone.
18. A phototherapeutic system, comprising:
   a base defining at least a portion of an irradiation zone; and
   a radiation assembly comprising ultraviolet (UV) source directed toward the irradiation zone, wherein-
      the UV source is configured to deliver a predetermined energy level during a phototherapy session,
      the radiation assembly is configured to deliver UV radiation within a predetermined wavelength spectrum, and
      the predetermined spectrum has a bandwidth of at most 16 nm and is focused at a wavelength corresponding to a maximum on a vitamin D3 phototherapy action spectrum for the predetermined energy level.
19. The phototherapeutic system of example 18 wherein the radiation assembly is focused at a wavelength of about 300-302 nm.
20. The phototherapeutic system of example 18 or 19 wherein the UV source comprises at least one LED focused at about 300-302 nm.

### IV. Conclusion

From the foregoing, it will be appreciated that specific embodiments of the technology have been described herein for purposes of illustration, but that various modifications may be made without deviating from the disclosure. Certain aspects of the new technology described in the context of particular embodiments may be combined or eliminated in other embodiments. Additionally, although advantages associated with certain embodiments of the new technology have been described in the context of those embodiments, other embodiments may also exhibit such advantages and not all embodiments need necessarily exhibit such advantages to fall within the scope of the technology. Accordingly, the disclosure and associated technology can encompass other embodiments not expressly shown or described herein.

## Claims

1. A method of determining the efficacy of a radiation assembly, the method comprising:
measuring irradiance data from a radiation assembly focused at a target wavelength;
multiplying irradiance values at a selected range of wavelengths between 280 nm and 320 nm with efficacy values of a vitamin D3 phototherapy action spectrum at the corresponding wavelengths to determine a weighted irradiance value at each wavelength, wherein the phototherapy action spectrum defines a wavelength having maximum vitamin D production per minimal erythemal dose at a predetermined energy level;
summing the weighted irradiance values to determine a total weighted irradiance value; and
dividing the total weighted irradiance value by a total of the irradiance values at the selected range of wavelengths to determine the efficiency of the radiation assembly; and
**characterised by** forming the vitamin D3 phototherapy action spectrum at the predetermined energy level, wherein forming the vitamin D3 phototherapy action spectrum comprises:
determining a percentage of photoproduct conversion for the predetermined energy level across a spectrum of wavelengths; and
multiplying the photoproduct conversion at a plurality of wavelengths with a ratio of CIE previtamin D3 production to CIE erythema action spectrum at the corresponding wavelengths, wherein the vitamin D3 phototherapy action spectrum for the predetermined energy level corresponds to a curve associated with the multiplied values at each wavelength.

2. The method of claim 1, further comprising:
measuring photoproduct conversion of a plurality of samples of 7-DHC exposed to the predetermined energy level at a corresponding plurality of wavelengths, wherein the photoproduct conversion measures quantities of previtamin D3, lumisterol, tachysterol, and 7-DHC in the samples of 7-DHC after exposure to the predetermined energy level; and
defining a photoisomerization action spectrum for the predetermined energy level, wherein the photoisomerization action spectrum defines the percentage of photoproduct conversion.

3. The method of claim 1 or 2, wherein the predetermined energy level is at most 1 J/cm².

4. The method of claim 1, 2 or 3, wherein the vitamin D3 phototherapy action spectrum is standardized by minimum erythemal dose.

5. The method of any of claims 1 to 4, further comprising a determining minimum erythemal dose of the radiation assembly by weighting irradiance values at a selected wavelength with a CIE erythema action spectrum at the selected wavelength.

6. The method of any of claims 1 to 5, wherein:
measuring irradiance data from the radiation assembly comprises measuring irradiance data for a plurality of radiation assemblies, each radiation assembly being focused at a different target wavelength; and
the method further comprises determining the efficiency of each radiation assembly by performing the steps of multiplying, summing and dividing for each radiation assembly.

7. The method of any of claims 1 to 6, wherein the target wavelength is between 300 nm and 302 nm.

8. The method of any of claims 1 to 7, wherein the radiation assembly comprises a metal halide lamp and a filter, the filter comprising an interference coating on a substrate, wherein the interference coating has a bandwidth of at most 16 nm.

## Patentansprüche

1. Verfahren zur Bestimmung der Wirksamkeit einer Strahlungsanordnung, wobei das Verfahren umfasst:
Messen von Bestrahlungsstärkedaten aus einer Strahlungsanordnung, die auf eine Zielwellenlänge fokussiert ist;
Multiplizieren von Bestrahlungsstärkewerten in einem ausgewählten Bereich von Wellenlängen zwischen 280 nm und 320 nm mit Wirksamkeitswerten eines Vitamin-D3-Phototherapie-Wirkungsspektrums bei den entsprechenden Wellenlängen, um einen gewichteten Bestrahlungsstärkewert bei jeder Wellenlänge zu bestimmen, worin das Phototherapie-Wirkungsspektrum eine Wellenlänge mit maximaler Vitamin-D-Produktion pro erythem wirksamer Mindestdosis bei einem vorbestimmten Energieniveau definiert;
Summieren der gewichteten Bestrahlungsstärkewerte, um einen gewichteten Bestrahlungsstärke-Gesamtwert zu bestimmen; und
Dividieren des gewichteten Bestrahlungsstärke-Gesamtwertes durch einen Gesamtbetrag der Bestrahlungsstärkewerte in dem ausgewählten Bereich von Wellenlängen, um die Effizienz der Strahlungsanordnung zu bestimmen; und
**gekennzeichnet durch** Ausbilden des Vitamin-D3-Phototherapie-Wirkungsspektrums bei dem vorbestimmten Energieniveau, worin das Ausbilden des Vitamin-D3-Phototherapie-Wirkungsspektrums umfasst:
Bestimmen eines Prozentsatzes der Fotoproduktumsetzung für das vorbestimmte Energieniveau über ein Spektrum von Wellenlängen; und
Multiplizieren der Fotoproduktumsetzung bei einer Mehrzahl von Wellenlängen mit einem Verhältnis von CIE-Prävitamin-D3-Produktion zu CIE-Erythem-Wirkungsspektrum bei den entsprechenden Wellenlängen, worin das Vitamin-D3-Phototherapie-Wirkungsspektrum für das vorbestimmte Energieniveau einer Kurve entspricht, die mit den multiplizierten Werten bei jeder Wellenlänge assoziiert ist.

2. Verfahren nach Anspruch 1, ferner umfassend:
Messen der Fotoproduktumsetzung einer Mehrzahl von Proben von 7-DHC, die dem vorbestimmten Energieniveau bei einer entsprechenden Mehrzahl von Wellenlängen ausgesetzt sind, worin die Fotoproduktumsetzung Mengen von Prävitamin D3, Lumisterol, Tachysterol und 7-DHC in den Proben von 7-DHC nach Exposition an das vorbestimmte Energieniveau misst; und
Definieren eines Fotoisomerisierungs-Wirkungsspektrums für das vorbestimmte Energieniveau, worin das Fotoisomerisierungs-Wirkungsspektrum den Prozentsatz der Fotoproduktumsetzung definiert.

3. Verfahren nach Anspruch 1 oder 2, worin das vorbestimmte Energieniveau höchstens 1 J/cm² beträgt.

4. Verfahren nach Anspruch 1, 2 oder 3, worin das Vitamin-D3-Phototherapie-Wirkungsspektrum mittels erythem wirksamer Mindestdosis standardisiert ist.

5. Verfahren nach einem der Ansprüche 1 bis 4, ferner umfassend das Bestimmen einer erythem wirksamen Mindestdosis der Strahlungsanordnung durch Gewichtung von Bestrahlungsstärkewerten bei einer ausgewählten Wellenlänge mit einem CIE-Erythem-Wirkungsspektrum bei der ausgewählten Wellenlänge.

6. Verfahren nach einem der Ansprüche 1 bis 5, worin:
das Messen von Bestrahlungsstärkedaten aus der Strahlungsanordnung das Messen von Bestrahlungsstärkedaten für eine Mehrzahl von Strahlungsanordnungen umfasst, wobei jede Strahlungsanordnung auf eine unterschiedliche Zielwellenlänge fokussiert ist; und
das Verfahren ferner das Bestimmen der Effizienz jeder Strahlungsanordnung durch Durchführung der Schritte des Multiplizierens, Summierens und Dividierens für jede Strahlungsanordnung umfasst.

7. Verfahren nach einem der Ansprüche 1 bis 6, worin die Zielwellenlänge zwischen 300 nm und 302 nm beträgt.

8. Verfahren nach einem der Ansprüche 1 bis 7, worin die Strahlungsanordnung eine Halogen-Metalldampflampe und einen Filter umfasst, wobei der Filter eine Interferenzbeschichtung auf einem Substrat umfasst, worin die Interferenzbeschichtung eine Bandbreite von höchstens 16 nm aufweist.

## Revendications

1. Procédé de détermination de l'efficacité d'un ensemble de radiation, le procédé consistant à :
mesurer des données d'irradiation en provenance d'un ensemble de radiation focalisé à une longueur d'onde cible ;
multiplier les valeurs d'irradiation à une plage sélectionnée de longueurs d'onde entre 280 nm et 320 nm par des valeurs d'efficacité d'un spectre d'action de photothérapie de vitamine D3 aux longueurs d'onde correspondantes pour déterminer une valeur d'irradiation pondérée à chaque longueur d'onde, le spectre d'action de photothérapie définissant une longueur d'onde ayant une production maximale de vitamine D par dose érythémale minimale à un niveau d'énergie prédéterminé ;
additionner les valeurs d'irradiation pondérées pour déterminer une valeur d'irradiation pondérée totale ; et
diviser la valeur d'irradiation pondérée totale par un total des valeurs d'irradiation à la plage sélectionnée de longueurs d'onde pour déterminer l'efficacité de l'ensemble de radiation ; et
le procédé étant **caractérisé par** l'étape consistant à :
former le spectre d'action de photothérapie de vitamine D3 au niveau d'énergie prédéterminé,
la formation du spectre d'action de photothérapie de vitamine D3 consistant à :
déterminer un pourcentage de conversion de photoproduits pour le niveau d'énergie prédéterminé dans un spectre de longueurs d'onde ; et
multiplier la conversion de photoproduits à une pluralité de longueurs d'onde par un rapport entre une production de prévitamine D3 CIE et un spectre d'action érythémale CIE aux longueurs d'onde correspondantes, le spectre d'action de photothérapie de vitamine D3 pour le niveau d'énergie prédéterminé correspondant à une courbe associée aux valeurs multipliées à chaque longueur d'onde.

2. Procédé selon la revendication 1, consistant en outre à :
mesurer une conversion de photoproduits d'une pluralité d'échantillons de 7-DHC exposés au niveau d'énergie prédéterminé à une pluralité correspondante de longueurs d'onde, la conversion de photoproduits mesurant des quantités de prévitamine D3, de lumistérol, de tachystérol et de 7-DHC dans les échantillons de 7-DHC après l'exposition au niveau d'énergie prédéterminé ; et
définir un spectre d'action de photoisomérisation pour le niveau d'énergie prédéterminé, le spectre d'action de photoisomérisation définissant le pourcentage de conversion de photoproduits.

3. Procédé selon la revendication 1 ou 2, dans lequel le niveau d'énergie prédéterminé est au plus de 1 J/cm².

4. Procédé selon la revendication 1, 2 ou 3, dans lequel le spectre d'action de photothérapie de vitamine D3 est standardisé par une dose érythémale minimale.

5. Procédé selon l'une quelconque des revendications 1 à 4, consistant en outre à déterminer une dose érythémale minimale de l'ensemble de radiation en pondérant des valeurs d'irradiation à une longueur d'onde sélectionnée avec un spectre d'action érythémale CIE à la longueur d'onde sélectionnée.

6. Procédé selon l'une quelconque des revendications 1 à 5, dans lequel :
la mesure de valeurs d'irradiation en provenance de l'ensemble de radiation consiste à mesurer des données d'irradiation pour une pluralité d'ensembles de radiation, chaque ensemble de radiation étant focalisé à une longueur d'onde cible différente ; et
le procédé consistant en outre à déterminer l'efficacité de chaque ensemble de radiation en réalisant les étapes de multiplication, addition et division pour chaque ensemble de radiation.

7. Procédé selon l'une quelconque des revendications 1 à 6, dans lequel la longueur d'onde cible est comprise entre 300 nm et 302 nm.

8. Procédé selon l'une quelconque des revendications 1 à 7, dans lequel l'ensemble de radiation comprenant une lampe aux halogénures métalliques et un filtre, le filtre comprenant un revêtement à interférence sur un substrat, le revêtement à interférence ayant une bande passante d'au plus 16 nm.
